# EUROPEAN PATENT APPLICATION

(11) **EP 2 194 118 A1**
(43) Date of publication of application: **09.06.2010**
(21) Application number: 08853899.6
(22) Date of filing: 18.11.2008
(51) Int. Cl.: C12N 1/20, A23C 9/127, A23L 1/30

(54) **AGENT AND METHOD FOR IMPROVING SURVIVABILITY OF LACTIC ACID BACTERIUM, AND FOOD COMPOSITION**

(30) Priority: 27.11.2007 JP 2007305585
(71) Applicant: Meiji Dairies Corporation, Koto-ku Tokyo 136-8908 (JP)
(72) Inventor: ISAWA, Kakuhei, Odawara-shi Kanagawa 250-0862 (JP); UCHIDA, Hideaki, Odawara-shi Kanagawa 250-0862 (JP); FURUICHI, Keisuke, Odawara-shi Kanagawa 250-0862 (JP); YAMAMOTO, Fuyuko, Odawara-shi Kanagawa 250-0862 (JP)
(74) Representative: Bentz, Jean-Paul
(86) International application number: PCT/JP2008/070889
(87) International publication number: WO 2009/069498

(57) **Abstract**

[Problem]

The survival of lactic acid bacterial strains such as probiotic bacteria contained in yogurt etc. is improved.

[Solving Means]

A lactic acid bacterium survival improver including a propionic acid bacterium fermentation product to improve the survival of a lactic acid bacterium. The propionic acid bacterium is a bacterium belonging to the genus Propionibacterium. The bacterium belonging to the genus Propionibacterium is Propionibacterium freudenreichii. Propionibacterium freudenreichii is Propionibacterium freudenreichii ET-3 (FERM BP-8115).

## Description

### [Technical Field]

The present invention relates to improvement in the survival of lactic acid bacterial strains contained in yogurt, etc., for example, probiotic bacteria, etc.

### [Background Art]

In recent years, bacterial strains having probiotic functions attract attention, and preventive medicine by alimentary canal improvement/normalization due to ingestion of these bacteria and by their accompanying immunoregulatory functions is becoming active. Particularly, lactic acid bacteria are also called probiotic bacteria and have been ingested as various foods from ancient times. Recently, their functional development is more actively pursued than ever before, and the development thereof as commercial products endowed with novel functionality is also advancing. Such bacterial strains include Lactobacillus gasseri and the like.

For ingestion of lactic acid bacteria, there are various foods, but fermented foods represented by fermented milk etc. are in low-pH environments that are not suitable for survival of lactic acid bacteria.

Because lactic acid bacteria have the probiotic functions as described above, improvement in the survival of lactic acid bacteria in foods and improvement in the survival of lactic acid bacteria after ingestion will contribute significantly to preventive medicine.

For improvement in the survival of lactic acid bacteria in foods and for improvement in the survival of lactic acid bacteria after ingestion, some methods have been proposed.

It is reported in Non-Patent Document 1 that in yogurt containing bifidobacteria and acidophilus bacteria, the maintenance of the number of bacteria during storage was improved by adding cysteine, whey powder, WPC, casein hydrates, or tryptone.

It is reported in Non-Patent Document 2 that the rise and fall of the number of probiotic bacteria (acidophilus bacteria) were improved by adding ascorbic acid as an active-oxygen scavenger.

On the other hand, Patent Document 1 discloses, as a "survival improver of a lactic acid bacterium", an improver containing a specific amount of lipids obtained by subjecting milk or milk materials (for example, butter serum, butter milk) to a specific treatment. Particularly, this improver is used by addition to a medium for lactic acid bacteria.

Patent Document 2 discloses a method of improving the survival of bifidobacteria, which includes using a culture of propionic acid bacteria and/or bacteria producing naphthoquinone ring-containing compounds, a solvent extract thereof, and/or its treated material. Particularly, it is disclosed that propionic acid bacteria produce a substance that promotes the growth of bifidobacteria.

In the prior art, improvement in survival was attempted by adding milk protein, an active-oxygen scavenger, etc. These substances significantly affect the flavor and physical properties of yogurt, and their use is limited. Particularly, an active-oxygen scavenger that is an antioxidant exhibits its effect through autoxidation, thus making it impossible to expect that a predetermined amount of the active-oxygen scavenger maintains its effect.

When milk or milk materials are used, their specific treatment is necessary and regulation therefor is troublesome, thus leading to an increase in costs.

It has already been known that when propionic acid bacteria etc. are used, a substance having a growth promoting effect on bifidobacteria is produced, but other working mechanisms are still not elucidated, and its effect on lactic acid bacteria is also unrevealed.

### [Non-Patent Document 1]

R. I. Dave and N. P. Shah, "Ingredient Supplementation Effects on Viability of Probiotic Bacteria in Yogurt", J. Dairy Sci. 81, 2804-2816 (1998)

### [Non-Patent Document 2]

Rajiv I. Dave and Negendra P. Shah, "Effectiveness of Ascorbic Acid as an Oxygen Scavenger in Improving Viability of Probiotic Bacteria in Yoghurts Made with Commercial Starter Cultures", Int. Dairy Journal 7, 435-443 (1997)

### [Patent Document 1]

JP-A 2007-097447

### [Patent Document 2]

JP-A 7-227207

### [Disclosure of the Invention]

### [Problem to be solved by the Invention]

An object of the present invention is to improve the survival of lactic acid bacterial strains.

Particularly, an object of the present invention is to improve the survival of lactic acid bacteria in foods represented by fermented milk etc. and also to improve the survival of lactic acid bacteria after ingestion.

Another object of the present invention is to improve the survival of a lactic acid bacterial strain contained in a food composition without influencing the flavor or physical properties of the food composition containing the lactic acid bacterial strain.

The lactic acid bacterium in the present invention can be exemplified by microorganisms belonging to the genus Lactobacillus and the genus Streptococcus, particularly by Lactobacillus gasseri whose excellent efficacy is being elucidated in recent years.

The "lactic acid bacterium" defined in the present invention refers to a bacterium that produces lactic acid in an amount of 50% or more based on milk sugar that was assimilated by the bacterium, and the "lactic acid bacterium" in the present invention does not encompass bifidobacteria.

### [Means for solving Problem]

For the purpose of improving the survival of a lactic acid bacterial strain contained in a food composition without influencing the flavor or physical properties of the food composition containing the lactic acid bacterial strain, the present inventors examined a method completely different from the method of improving the survival or viability of lactic acid bacteria by addition of an antioxidant etc. as shown in the prior art.

As a result, the present inventors found that by using a fermentation product (culture) of propionic acid bacteria, the maintenance of lactic acid bacteria contained in a food composition is elevated and the qualities thereof during storage can be maintained without influencing the flavor and physical properties of the food composition containing the lactic acid bacterial strain, and the present invention was thereby completed.

The invention described in claim 1 is directed to a survival improver of a lactic acid bacterium, which includes a propionic acid bacterium fermentation product.

The invention described in claim 2 is directed to the survival improver of a lactic acid bacterium according to claim 1, wherein the propionic acid bacterium is a bacterium belonging to the genus Propionibacterium.

The invention described in claim 3 is directed to the survival improver of a lactic acid bacterium according to claim 2, wherein the bacterium belonging to the genus Propionibacterium is Propionibacterium freudenreichii.

The invention described in claim 4 is directed to the survival improver of a lactic acid bacterium according to claim 3, wherein Propionibacterium freudenreichii is Propionibacterium freudenreichii ET-3 (FERM BP-8115).

The invention described in claim 5 is directed to the survival improver of a lactic acid bacterium according to any of claims 1 to 4, wherein the lactic acid bacterium is at least one member selected from Lactobacillus gasseri, Lactobacillus bulgaricus, and Streptococcus thermophilus.

The invention described in claim 6 is directed to a method of improving the survival of a lactic acid bacterium, which includes adding the survival improver of a lactic acid bacterium according to any of claims 1 to 5 to a lactic acid bacterium-containing composition.

The invention described in claim 7 is directed to the method of improving the survival of a lactic acid bacterium according to claim 6, wherein before fermentation of the lactic acid bacterium-containing composition, the survival improver of a lactic acid bacterium is added to the lactic acid bacterium-containing composition.

The invention described in claim 8 is directed to a food composition containing the survival improver of a lactic acid bacterium according to claim 1 and a lactic acid bacterium.

### [Effect of the Invention]

According to the present invention, the survival of a lactic acid bacterial strain can be improved. According to the present invention, the survival of lactic acid bacteria in fermented foods represented by fermented milk etc. can be improved, and the survival of lactic acid bacteria after ingestion can be improved.

According to the present invention, the survival of a lactic acid bacterial strain contained in a food composition can be improved without influencing the flavor and physical properties of the lactic acid bacterial strain-containing food composition.

According to the present invention, the survival of lactic acid bacteria of the genus Lactobacillus and the genus Streptococcus, etc., particularly Lactobacillus gasseri whose excellent efficacy is being elucidated in recent years can be improved in food compositions containing the lactic acid bacterial strains without influencing the flavor and physical properties thereof.

Accordingly, there can be provided the intended foods etc. that can be endowed with high probiotic functions even after long storage without deteriorating the original flavor and physical properties of the foods.

### [Best Mode for carrying out the Invention]

The survival improver of a lactic acid bacterium proposed in the present invention includes a fermentation product of a propionic acid bacterium.

That is, the present invention is to improve the survival of lactic acid bacterial strains such as probiotic bacteria contained in yogurt etc. by using a fermentation product of a propionic acid bacterium. The objects described above could be achieved by providing a survival improver of a lactic acid bacterium, which includes a fermentation product of a propionic acid bacterium.

A propionic acid bacterium has been used as a starter for Emmentaler cheese etc. for a long time, and its safety has been confirmed.

The fermentation product of a propionic acid bacterium used in the survival improver of a lactic acid bacterium proposed in the present invention is obtained by culturing a propionic acid bacterium in a usual manner as described below.

In this specification, the "fermentation product of a propionic acid bacterium" refers to a culture itself obtained by fermentation with a propionic acid bacterium.

The survival improver of a lactic acid bacterium, which includes a fermentation product of a propionic acid bacterium, according to the present invention includes a fermentation product of a propionic acid bacterium and its treated materials, for example, a culture filtrate or a culture supernatant obtained from the above culture (propionic acid bacterium fermentation product) by eliminating the bacteria through filtration, centrifugation or membrane separation, etc., a concentrate obtained by concentrating the culture filtrate, the culture supernatant or the propionic acid bacterium fermentation product, etc., with an evaporator etc., its paste material, diluted material or dried material (obtained by lyophilization or heating under reduced pressure, etc.), or their disinfected or sterilized materials.

In preparation of the treated material in the present invention, the treatment steps such as bacteria elimination treatments such as filtration, centrifugation and membrane separation, as well as precipitation, concentration, formation into paste, dilution, drying, disinfection, sterilization etc. can be used alone or in combination thereof.

In the present invention, the fermentation product and its treated material refer to those not subjected to heat treatment.

The medium used in production of the propionic acid bacterium fermentation product usually contain nutrient sources with which microorganisms can grow. The nutrient sources that can be added include whey, casein, powdered skim milk, whey protein concentrate (WPC), whey protein isolate (WPI), yeast extract, soybean extract, peptone such as trypticase, etc., sugars such as glucose and lactose, etc., and minerals such as whey mineral, etc., or foods containing them. Alternatively, enzymatically treated materials thereof may be added. Particularly, whey or an enzymatically treated material thereof is preferably used, to which the nutrient source is however not limited. In this specification, the propionic acid bacterium fermentation product using whey or a whey-derived material such as WPC or WPI as a nutrient source is hereinafter referred to as a propionic acid bacterium whey fermentation product.

The enzyme used in enzymatically decomposing the nutrient source includes one of proteases or peptidases or a combination thereof. The proteases or peptidases used are not particularly limited. For example, food-grade proteases include an endoprotease, an exoprotease, an exopeptidase/endoprotease-conjugated enzyme and a protease/peptidase-conjugated enzyme. The endoprotease includes, for example, chymosin (EC 3.4.23.4, Maxiren, derived from modified yeast Kluyveromyces lactis, GIST-BROCADES N.V.), Alcalase R (derived from Bacillus licheniformis, Novo), Esperase (derived from B. lentus, Novo), Neutrase R (derived from B. subtilis, Novo), Protamex (derived from bacteria, Novo), PTN6.0S (porcine pancreatic trypsin, Novo), etc. The exopeptidase/endoprotease-conjugated enzyme includes, for example, Flavourzyme (derived from Aspergillus oryzae, Novo) etc. Other endoproteases include, for example, trypsin (CAS No. 9002-07-7, EC 3.4.21.4, derived from bovine pancreas, Product No. T8802, SIGMA), pepsin (CAS No. 9001-75-6, EC 3.4.4.1, derived from porcine gastric mucosa, SIGMA), chymotrypsin (Novo, Boehringer), Protease N "Amano" G (derived from Bacillus subtilis, Amano Enzyme Inc.), Bioprase (derived from Bacillus subtilis, Nasase & CO., LTD), and Papain W-40 (Amano Enzyme Inc.). The exoprotease includes a pancreatic carboxypeptidase, an aminopeptidase of an intestinal brush border, etc. The protease/peptidase-conjugated enzyme includes, for example, Protease A "Amano" G (derived from Aspergillus oryzae, Amano Enzyme Inc.) and Umamizyme G (peptidase and protease, derived from Aspergillus oryzae, Amano Enzyme Inc.). Origins of the enzymes are not limited to those described above, and the enzymes may be derived from animals, plants and microorganisms, but are preferably those derived from Aspergillus oryzae. These enzymes are not intended to be those limited by trade names, origins, manufacturers etc. In carrying out the present invention, the enzymes may be used alone or in combination of two or more thereof. A preferable example includes, but is not limited to, Protease A "Amano" G (derived from Aspergillus oryzae, Amano Enzyme Inc.). When a combination of these enzymes is used, their respective enzyme reactions may be carried out simultaneously or separately.

When the nutrient source is decomposed with the enzyme, the nutrient source as a starting material is dispersed and dissolved in water or hot water and then decomposed by adding the enzyme. The pH at the start of the enzyme decomposition, the enzyme decomposition time and the enzyme reaction temperature are not particularly limited as long as the product of the present invention can be obtained. These conditions can be described as follows: The pH at the start of the enzyme decomposition is 3.0 to 7.5, preferably 4.5 to 7.0, more preferably 6.0 to 7.0. The enzyme decomposition time is 0.5 to 300 hours, preferably 1 to 20 hours, more preferably 1 to 5 hours. The enzyme reaction temperature is 20 to 57°C, preferably 30 to 52°C, more preferably 40 to 50°C.

A preferable example of preparation of a medium for producing the propionic acid bacterium fermentation product includes, but is not limited to, a method wherein whey powder (10 w/w%) and Protease Amano A "Amano" G (0.07 w/w%, Amano Enzyme Inc.) are dissolved in water and then subjected to enzyme decomposition at 47°C, pH 6.6, for 2 hours and then heated at 85°C for 10 minutes thereby deactivating the enzyme, then beer yeast extract (0.10 w/w%, manufactured by Asahi Breweries, Ltd.) and ammonium sulfate (0.27 w/w%) are added thereto, and the pH was adjusted to 6.7, followed by sterilization at 121°C for 7 minutes.

The propionic acid bacteria used in production of the propionic acid bacterium fermentation product include those belonging to the genus Propionibacterium, the genus Propionicimonas, the genus Propioniferax, the genus Propionimicrobium, the genus Propionivibrio etc.; the bacteria are not particularly limited, but are preferably those belonging to the genus Propionibacterium.

Examples of bacteria of the genus Propionibacterium include bacteria for cheese, such as Propionibacterium freudenreichii, Propionibacterium thoenii, Propionibacterium acidipropionici, and Propionibacterium jensenii, etc., as well as Propionibacterium avidum, Propionibacterium acnes, Propionibacterium lymphophilum, Propionibacterium granulosam, Propionibacterium arabinosum, Propionibacterium cyclohexanicum, Propionibacterium innocuum, Propionibacterium intermediu, Propionibacterium pentosaceum, Propionibacterium peterssonii, Propionibacterium propionicum, and Propionibacterium zeae, etc., among which Propionibacterium freudenreichii (also referred to hereinafter as P. freudenreichii) is preferable, Propionibacterium freudenreichii IFO 12424 or Propionibacterium freudenreichii ATCC 6207 is more preferable, and Propionibacterium freudenreichii ET-3 (FERM BP-8115) is particularly preferable.

The present inventors deposited Propionibacterium freudenreichii ET-3 with International Patent Organism Depositary, National Institute of Advanced Industrial Science and Technology, Japan. This deposition is specifically as follows:

(1) Depository institution: International Patent Organism Depositary (IPOD), National Institute of Advanced Industrial Science and Technology (AIST)
(2) Address: Central 6, Higashi 1-1-1, Tsukuba City, Ibaraki Pref. (zip code: 305-8566) Japan
(3) Accession Number: FERM BP-8115
(4) Indication for identification: ET-3
(5) Date of original deposition: August 9, 2001
(6) Date of transfer to deposition under the Budapest Treaty: July 11, 2002
   Propionibacterium freudenreichii ET-3 mentioned above was separated from Emmentaler cheese. This bacterium is a Gram-positive short bacillus whose nitrate reduction ability is negative; indole production ability is negative; hydrogen sulfide production ability is negative; and lactose fermentation ability is positive. This bacterium also has an ability to produce 1.4-dihydroxy-naphthoic acid (DHNA) .

A bacterial strain having a strain name designated ATCC refers to a standard strain obtained from American Type Culture Collection (ATCC), and a bacterial strain having a strain name designated IFO refers to a standard strain obtained from Institute for Fermentation, Osaka (IFO).

Then, this propionic acid bacterium is cultured in the medium. Although the culture method that can be used may be an anaerobic or aerobic culture method known in the art, an anaerobic or aerobic culture method with a liquid medium is preferably used. Although the pH at the start of fermentation, the fermentation time and the fermentation temperature are not particularly limited as long as the survival improver of lactic acid bacteria according to the present invention can be obtained, the pH at the start of fermentation is 3.0 to 7.5, preferably 5.0 to 7.5, more preferably pH 5.5 to 7.0. The fermentation time is 0.5 to 200 hours, preferably 50 to 100 hours, more preferably 60 to 99 hours, and the fermentation temperature is 20 to 50°C, preferably 25 to 45°C, more preferably 30 to 40°C. The culture obtained in this manner may be used immediately after culturing, but desirably the culture is used after cooling (3 to 20°C, preferably about 10°C) and stored for about 2 to 4 weeks. A preferable example includes, but is not limited to, a method where a medium adjusted to pH 6.8 and sterilized at 121°C for 7 minutes is inoculated with 0.01 to 2.5% activated culture of P. freudenreichii ET-3 (FERM BP-8115) which is then anaerobically cultured at 32 to 37°C for 72 to 99 hours in a nitrogen atmosphere, and its culture is obtained as a propionic acid bacterium fermentation product.

For example, if a medium prepared by decomposing about 10 w/w% aqueous whey solution with Protease A "Amano" G (at 40 to 52°C, pH 6.0 to 7.0, for 1 to 5 hours) and then adding beer yeast extract and ammonium sulfate thereto is inoculated with 0.01 to 2.5% activated culture of Propionibacterium freudenreichii ET-3 described above which is then anaerobically cultured at 32 to 37°C for 72 to 99 hours in a nitrogen atmosphere to produce a propionic acid bacterium whey fermentation product, then the bacterial density of Propionibacterium freudenreichii ET-3 is about 0.5 to 50×cfu/mL, the content of DHNA is about 5 to 500 µg/mL, and the solid content is about 5 to 15 w/w%.

The survival improver of lactic acid bacteria according to the present invention can also be obtained as the propionic acid bacterium fermentation product or its treated material as it is or as a soluble or insoluble fraction diluted with a solvent. As the solvent, water or ordinarily used solvents such as alcohols, hydrocarbons, organic acids, organic bases, inorganic acids, inorganic bases, supercritical fluids etc. can be used alone or in combination of two or more thereof.

The propionic acid bacterium fermentation product itself and/or its treated material may be formed into food compositions combined with water, proteins, carbohydrates, lipids, vitamins, minerals, organic acids, organic bases, fruit juices, flavors etc.

According to the inventor's examination, the survival improver of lactic acid bacteria according to the present invention could exhibit a function in improving the survival of lactic acid bacteria in low-pH environments. Lactic acid bacterium-containing foods represented by fermented milk etc. are generally in low-pH environments and originally not suitable for survival of lactic acid bacteria. The survival improver of the present invention can exhibit a function in improving the survival of lactic acid bacteria in low-pH environments and is thus useful in improving the survival of lactic acid bacteria in foods represented by fermented milk etc. containing lactic acid bacteria. In consideration of the pH of lactic acid bacterium-containing foods, the low-pH environment under which the survival improver of lactic acid bacteria according to the present invention can be more efficiently exhibited is preferably at pH 2.5 to 6.5, more preferably pH 3.5 to 5,5, even more preferably pH 3.8 to 4.8.

The lactic acid bacterial strains, particularly probiotic bacteria, whose survival could be confirmed to be improved by the survival improver of lactic acid bacteria proposed in the present invention can be exemplified by microorganisms belonging to the genus Lactobacillus and the genus Streptococcus.

The microorganisms whose survival could be confirmed to be improved by the survival improver of lactic acid bacteria according to the present invention were for example Lactobacillus gasseri, Lactobacillus bulgaricus, Streptococcus thermophilus, particularly Lactobacillus bulgaricus JCM 1002^{T}, Lactobacillus bulgaricus OLL1255 (NITE P-76), Streptococcus thermophilus ATCC 19258^{T}, Streptococcus thermophilus OLS3294 (NITE P-77), and Lactobacillus gasseri OLL2716 (FERM BP-6999).

Even if these bacterial strains are used alone or in combination of two or more thereof, their survival could be confirmed to be improved by the survival improver of lactic acid bacteria according to the present invention.

Information on the deposition of Lactobacillus bulgaricus OLL1255 (NITE P-76), Streptococcus thermophilus OLS3294 (NITE P-77) and Lactobacillus gasseri OLL2716 (FERM BP-6999) is as follows:

Lactobacillus bulgaricus OLL1255 (NITE P-76)
(1) Depository institution: NITE Patent Microorganisms Depositary (NPMD), National Institute of Technology and Evaluation (NITE)
(2) Address: Nishihara 2-49-10, Shibuya Ward, Tokyo (zip code: 151-0066) Japan
(3) Accession Number: NITE P-76
(4) Indication for identification: Lactobacillus bulgaricus OLL1255 (NITE P-76)
(5) Date of original deposition: February 10, 2005

Streptococcus thermophilus OLS3294 (NITE P-77)
(1) Depository institution: NITE Patent Microorganisms Depositary (NPMD), National Institute of Technology and Evaluation (NITE)
(2) Address: Nishihara 2-49-10, Shibuya Ward, Tokyo (zip code: 151-0066) Japan
(3) Accession Number: NITE P-77
(4) Indication for identification: Streptococcus thermophilus OLS3294
(5) Date of original deposition: February 10, 2005

Lactobacillus gasseri OLL2716 (FERM BP-6999)
(1) Depository institution: National Institute of Bioscience and Human-Technology, Agency of Industrial Science and Technology, Ministry of International Trade and Industry
(2) Address: Higashi 1-1-3, Tsukuba City, Ibaraki Pref. (zip code: 305-8566) Japan
(3) Accession Number: FERM BP-6999
(4) Indication for identification: Lactobacillus gasseri OLL2716
(5) Date of original deposition: May 24, 1999
(6) Date of transfer to deposition under the Budapest Treaty: January 14, 2000

The method of improving the survival of lactic acid bacteria according to the present invention is carried out by adding the propionic acid bacterium fermentation product-containing survival improver of lactic acid bacteria to a lactic acid bacterium-containing composition. The survival improver may be added before, during or after fermentation of a lactic acid bacterium-containing composition, preferably before fermentation of the composition.

According to the present invention, the survival improver of the present invention can also be used in a food composition. That is, the food composition contains the survival improver and the lactic acid bacterium.

The food composition includes various drinks and foods (soft drink, fermented drink, yogurt etc.).

The food composition containing the survival improver and the lactic acid bacterium according to the present invention can be used as it is or may be used in a usual manner for usual food compositions by mixing it with other foods or food components. With respect to other forms, the food composition of the present invention may be in usual forms such as those of ordinary drinks and foods, for example in any forms of solids (powders, granules etc.), pastes, liquids and suspensions.

Other components are not particularly limited, and water, proteins, carbohydrates, lipids, vitamins, minerals, organic acids, organic bases, fruit juices, flavors etc. can be used as components in the food composition. These components can be used as a combination of two or more thereof, and synthetic products and/or foods containing them in a large amount may also be used.

Hereinafter, the present invention is described in more detail by reference to the Examples, but the present invention is not limited thereto.

### Examples

The influence of the lactic acid bacterium survival improver of the present invention on the survival and low-pH growth activity of Lactobacillus gasseri OLL2716 that is a probiotic bacterial strain in Yogurt LG21 (manufactured by Meiji Dairies Corporation) was examined.

A yogurt material consisting of cow milk, powdered skimmed milk and a sweetener was satirized by heating. Then, concentrated starters (2% by weight of Lactobacillus bulgaricus OLL1255, 2% by weight of Streptococcus thermophilus OLS3294 and 0.05% by weight of Lactobacillus gasseri OLL2716), and 0.5% by weight of a propionic acid fermentation product concentrated until the concentration of 1,4-dihydroxy-2-naphthoic acid (DHNA) had reached 100 µg/ml, were simultaneously added to and mixed with the yogurt material and then fermented at 43°C until the acidity of lactic acid reached 0.75%, to prepare yogurt.

Separately, yogurt was prepared as comparative yogurt without adding the propionic acid fermentation product.

The propionic acid bacterium fermentation product described above was prepared in the following manner.

180 g of powdered skimmed milk (manufactured by Meiji Dairies Corporation) was dissolved in 1000 g of water and regulated at a temperature of 47°C. 3.75 g of a protease was added to this solution followed by protein decomposition at 47°C for 3 hours. During protein decomposition, the pH was regulated at 6.6 to 6.8 with an aqueous solution of potassium carbonate.

After protein decomposition, the solution was heated to 80°C and kept at this temperature for 10 minutes thereby inactivating the protease, followed by adding 7.5 g of beer yeast extract and 15 g of milk sugar and adjusting the pH to 6.95 with an aqueous solution of potassium carbonate.

The solution was adjusted in volume to 1500 mL with water, then introduced into a 2 L fermenter and sterilized as a medium (concentration of sugar milk: about 6.1 %). The sterilization conditions were 121°C and 7 minutes.

After sterilization, the fermenter was aerated with nitrogen gas, and the medium temperature was stabilized at 33°C, and 0.75 mL of a frozen concentrated starter (P. freudenreichii ET-3) was added to the medium to initiate culturing. During culturing, the culture medium was regulated at a temperature of 33°C and at pH 6.5 and aerated with nitrogen gas. For regulation of the pH, 40% (wt/wt) aqueous solution of potassium carbonate was used.

72 hours after culturing was initiated, aeration with nitrogen gas was changed to aeration with air, and 168 hours after culturing was initiated, culturing was terminated. The flow rate of air during aeration was 2 L/min, and the stirring rate was 150 rpm.

As a result, a culture (propionic acid fermentation product) containing DHNA at a concentration of 52 µg/mL was obtained. After 72 hours, the concentration of milk sugar was about 1.9% by mass, and the number of propionic acid bacteria was 3.5×10¹⁰ cfu/mL.

The amount of DHNA in the culture (propionic acid fermentation product) was determined as follows: 0.1% (w/v) sodium ascorbate was added to 10 ml of the culture which was then adjusted to pH 7.0 and adjusted to a volume of 20 ml with water, and 3 ml of its aliquot was mixed with an equal volume of methanol and then centrifuged at 3000 rpm for 10 minutes, and its supernatant was filtered through a 0.45 µm filter and subjected to HPLC. The amount of DHNA in the sample was determined from the relationship between the peak area in HPLC and DHNA concentrations (in a calibration curve) and from the predetermined retention time (in the vicinity of 13 minutes) of a commercial DHNA standard (manufactured by Wako Pure Chemical Industries, Ltd.).

The yogurts obtained above were stored at 10°C for a period of 16 days that is the expiration date of usual commercial yogurt products, and then a change in the number of bacteria of each lactic acid bacterial strain and a transition in the low-pH growth ability of the OLL2716 strain were examined.

As a container of the yogurt, a polystyrene cup and an aluminum lid were used.
The number of propionic acid bacteria in the yogurt was measured by anaerobic culture in YEL (Yeast Extract Lactate) agar medium at 30°C. The number of bacteria was kept at about 1×10⁸ cfu/g just in the yogurt just after preparation (fresh product) until after 16-day storage.

The survival rate of the OLL2716 strain after 16-day storage, based on that in the fresh product, was 7.8% in the comparative example and 50.8% in the example.

The survival rate of the OLS3294 strain after 16-day storage, based on that in the fresh product, was 7.5% in the comparative example and 73.6% in the example.

The survival rate of the OLL1255 strain after 16-day storage, based on that in the fresh product, was 50% in the comparative example and 98% in the example.

The low-pH growth activity was expressed by diluting each sample 10-fold, then inoculating 100 µl of the resulting dilution into 5 ml of MRS-broth (pH 4.5), culturing the strain at 37°C for 6 hours under aerobic conditions, and then determining the growth ratio of the OLL2716 strain.

At this time, the low-pH growth activity was 2.6-fold in the comparative example and 3.5-fold in the example after a lapse of 1 day, or 0.9-fold in the comparative example and 1.8-fold in the example after a lapse of 16 days.

The influence of addition of the propionic acid fermentation product on the flavor and physical properties of the yogurt was recognized neither in the fresh product nor in the product after 16-day storage.

The examination in the example described above was conducted by concentrating the propionic acid bacterium fermentation product prepared as described above to a DHNA concentration of 100 µg/ml and then adding 0.5% by weight of the concentrate; instead, a similar examination was conducted by changing the amount of the propionic acid bacterium fermentation product added.

As a result, it was confirmed that the amount of the propionic acid bacterium fermentation product added to the lactic acid bacterium-containing composition is desirably 0.1 to 10% by weight, preferably 0.3 to 5% by weight, from the viewpoint of the survival and viability (activity) of the probiotic bacterium (Lactobacillus gasseri) during chilled storage.

From the forgoing results, the survival and viability (activity) of the probiotic bacterium (Lactobacillus gasseri) during chilled storage was significantly improved by adding the propionic acid bacterium fermentation product to the yogurt. The survival of the thermophilus bacteria used as a basic starter was also similarly improved.

Because the survival of the propionic acid bacteria in yogurt is high, it is estimated that the effect of the present invention is sufficiently persistently maintained within the expiration date (about 25 days) of yogurt.

### [Industrial Applicability]

By using the survival improver of lactic acid bacteria according to the present invention, the survival of various lactic acid bacteria, particularly lactic acid bacteria of the genus Lactobacillus, particularly Lactobacillus gasseri, etc., and the genus Streptococcus, etc., can be improved. There can also be provided foods etc. which can be endowed with probiotic functions even after long storage without deteriorating the original flavor and physical properties of the foods.

## Claims

1. A survival improver of a lactic acid bacterium, which comprises a propionic acid bacterium fermentation product.

2. The survival improver of a lactic acid bacterium according to claim 1, wherein the propionic acid bacterium is a bacterium belonging to the genus Propionibacterium.

3. The survival improver of a lactic acid bacterium according to claim 2, wherein the bacterium belonging to the genus Propionibacterium is Propionibacterium freudenreichii.

4. The survival improver of a lactic acid bacterium according to claim 3, wherein Propionibacterium freudenreichii is Propionibacterium freudenreichii ET-3 (FERM BP-8115).

5. The survival improver of a lactic acid bacterium according to any of claims 1 to 4, wherein the lactic acid bacterium is at least one member selected from Lactobacillus gasseri, Lactobacillus bulgaricus, and Streptococcus thermophilus.

6. A method of improving the survival of a lactic acid bacterium, **characterized in that** the method comprises adding the survival improver of a lactic acid bacterium according to any of claims 1 to 5 to a lactic acid bacterium-containing composition.

7. The method of improving the survival of a lactic acid bacterium according to claim 6, **characterized in that** before fermentation of the lactic acid bacterium-containing composition, the survival improver of a lactic acid bacterium is added to the lactic acid bacterium-containing composition.

8. A food composition comprising the survival improver of a lactic acid bacterium according to claim 1 and a lactic acid bacterium.
